# EUROPÄISCHE PATENTSCHRIFT

(11) **EP 3 402 774 B1**
(45) Veröffentlichungstag und Bekanntmachung des Hinweises auf die Patenterteilung: **12.02.2020**
(21) Anmeldenummer: 17712440.1
(22) Anmeldetag: 16.03.2017
(51) Int. Cl.: C07C 29/152, C07C 31/04, B01J 19/18

(54) **VERFAHREN MIT EINEM REAKTOR MIT KÜHLEINRICHTUNG**
PROCESS WITH A REACTOR WITH COOLING DEVICE
PROCÉDÉ AVEC UN RÉACTEUR À DISPOSITIF DE REFROIDISSEMENT

(30) Priorität: 22.03.2016 DE 102016204718
(43) Veröffentlichungstag der Anmeldung: 21.11.2018
(73) Patentinhaber: Siemens Aktiengesellschaft, 80333 München (DE)
(72) Erfinder: ALBERT, Jakob, 91054 Erlangen (DE); BALDAUF, Manfred, 91056 Erlangen (DE); REICHERT, Jenny, 97523 Schwanfeld (DE); STARK, Katharina, 91052 Erlangen (DE); TREMEL, Alexander, 91096 Möhrendorf (DE); WASSERSCHEID, Peter, 91054 Erlangen (DE)
(86) Internationale Anmeldenummer: PCT/EP2017/056247
(87) Internationale Veröffentlichungsnummer: WO 2017/162513

(56) Entgegenhaltungen:
- WO-A1-2015/030578
- DE-B- 1 301 524
- US-A- 5 712 313
- US-A1- 2003 086 853
- US-A1- 2007 248 849

## Beschreibung

Die Erfindung betrifft ein Verfahren zum Betreiben eines Reaktors nach Patentanspruch 1 und eine Verwendung nach Patentanspruch 5.

Der Umsatz bei chemischen Reaktionen ist durch die Gleichgewichtslage der Reaktion beschränkt. Liegt das chemische Gleichgewicht einer Synthesereaktion nur teilweise auf der Seite der Produkte, führt eine einstufige Reaktionsführung nur zu einem Teilumsatz. Werden dagegen kontinuierlich die Reaktionsprodukte aus dem Reaktor abgeführt, findet im Reaktor ein kontinuierlicher Umsatz von Edukten zu Produkten statt. Zur kontinuierlichen Abfuhr von Reaktionsprodukten können Sorptionsmittel verwendet werden. Diese Sorptionsmittel bilden eine zusätzliche Phase die Selektivprodukte aufnimmt, die dadurch aus dem chemischen Gleichgewicht entfernt werden. Die Sorptionsphase kann mitsamt dem Produkt aus dem Reaktor ausgeschleust werden. US-A-5 712 313 offenbart ein Verfahren mit einem Sorptionsmittel.

Die Aufgabe der Erfindung besteht darin, ein Verfahren bereitzustellen, die dafür geeignet ist, gleichgewichtslimitierte Reaktionen unter Verwendung eines Sorptionsmittels durchzuführen und dabei gegenüber den Stand der Technik, die Ausbeute an Reaktionsprodukten zu erhöhen.

Die Lösung der Aufgabe besteht in einem Verfahren mit den Merkmalen des Patentanspruchs 1.

Der nicht beanspruchte Reaktor weist einen Reaktionsraum auf, wobei am Reaktionsraum eine Zuführvorrichtung für Edukte, eine Zuführvorrichtung für flüssiges Sorptionsmittel sowie eine Ablassvorrichtung für das Sorptionsmittel angeordnet sind. Dabei befindet sich in einem unteren Bereich des Reaktionsraums eine Sorptionsmittelsammelzone, wobei sich der Reaktor dadurch auszeichnet, dass eine Kühlvorrichtung zur Kühlung des Sorptionsmittels vorgesehen ist.

Die Kühlung des Sorptionsmittels bewirkt zum einen, dass einer thermischen Zersetzung des Sorptionsmittels vorgebeugt wird, was wiederum die Auswahl an möglichen Sorptionsmitteln erhöht und den Einsatz effizienterer Sorptionsmittel bei der Reaktion erlaubt. Andererseits wird durch die Kühlung des Sorptionsmittels die Erwärmung im gesamten Reaktionsraum reduziert, was sich positiv auf den Gleichgewichtsumsatz der Reaktion auswirkt.

Die Kühlvorrichtung ist im Bereich der Sorptionsmittelsammelzone angeordnet. Dies führt zu einer effektiven Kühlung des Sorptionsmittels und zu einer guten Wärmeübertragung zwischen der Kühlvorrichtung und dem flüssigen Sorptionsmittelmedium.

Wiederum ist dabei die Kühlvorrichtung so angeordnet, dass sie in einem Betriebszustand unterhalb oder zumindest teilweise eines Flüssigkeitsspiegels des Sorptionsmittels liegt. Durch diese Maßnahme ist eine optimale Wärmeübertragung von der Kühlvorrichtung zum Sorptionsmittel möglich.

Zwischen der Ablassvorrichtung und der Zuführvorrichtung ist eine Rezykliervorrichtung zur Rezirkulation des Sorptionsmittels angeordnet. Diese Rezykliervorrichtung ist dazu geeignet, das Sorptionsmittel erneut in den Reaktionsraum einzubringen, wenn es dabei bereits gekühlt ist, wirkt sich dies auch positiv auf die Stabilität während der Reaktion aus.

Ferner ist es zweckmäßig, in der Sorptionsmittelsammelzone eine Umwälzvorrichtung, insbesondere eine Rührvorrichtung anzuordnen, um den Austausch von Wärme zwischen dem Sorptionsmittel und der Kühlvorrichtung zu gewährleisten.

Ferner ist es zweckmäßig, zur Beschleunigung der Umsetzungsreaktion, im Reaktionsraum einen Katalysatorraum zur Aufnahme eines Katalysators anzuordnen. Der Katalysator sorgt dafür, dass die Reaktion schneller abläuft, er beeinflusst jedoch nicht im Wesentlichen den Gleichgewichtszustand. Reaktionsprodukte, die an der Katalysatoroberfläche stehen, werden vom Sorptionsmittel absorbiert und aus dem Reaktionsraum geführt.

Dabei ist es zweckmäßig, wenn der Reaktionsraum den Katalysatorraum und einen Sorptionsraum umfasst. Eine Trennung des Katalysatormaterials vom Sorptionsmittel ist bei vielen Paarungen von Katalysator und Sorptionsmittel zweckmäßig, da beide Stoffe sich bezüglich der Reaktion negativ beeinflussen können.

Dabei ist es wiederum zweckmäßig, dass der Sorptionsraum und der Katalysatorraum durch ein gasdurchlässiges Element voneinander getrennt sind. Dieses Element ist dabei wiederum zweckmäßigerweise für Flüssigkeiten bzw. für Flüssigkeitstropfen undurchlässig. Auf dieser Weise kann ein gasförmiges Reaktionsprodukt leichter vom Katalysatorraum in den Sorptionsraum gelangen, ohne dass dabei Sorptionsmittel, das in flüssiger Form vorliegt, in den Katalysatorraum gelangen kann. Bei dem Element kann es sich beispielsweise um ein Gewebe, insbesondere ein metallisches Gewebe oder um eine selektive Membran handeln.

Ferner ist es zweckmäßig, wenn der Reaktor einen Phasenabscheider aufweist, der außerhalb des Reaktionsraumes angeordnet ist und der dazu dient, eine Trennung zwischen Sorptionsmittel und dem Reaktionsprodukt vorzunehmen. Dabei steht dieser Phasenabscheider mit der Ablassvorrichtung des Reaktors in Verbindung. Dieser Phasenabscheider ist dabei in bevorzugter Weise Bestandteil der Rezykliervorrichtung.

Bestandteil der Erfindung ist ein Verfahren zum Betreiben eines Reaktors zur Umsetzung gleichgewichtslimitierter Reaktionen mit den Merkmalen des Patentanspruchs 1.

Dieses Verfahren umfasst dabei folgende Schritte: Zum einen werden mehrere Reaktionsedukte in einen Reaktionsraum eingebracht. Des Weiteren wird ebenfalls in den Reaktionsraum ein flüssiges Sorptionsmittel eingeführt. Die Edukte werden dabei an einen im Reaktionsraum befindlichen Katalysator geführt, wobei dieser dort an der Katalysatoroberfläche bis zum Auftreten einer Gleichgewichtssituation zu Reaktionsprodukten umgesetzt werden. Die Reaktionsprodukte werden von der Katalysatoroberfläche zum Sorptionsmittel geführt und von diesem absorbiert wonach sich das mit dem Reaktionsprodukt beladene Sorptionsmittel in einer Sorptionsmittelsammelzone absetzt. Die Erfindung zeichnet sich dadurch aus, dass das flüssige Sorptionsmittel im Reaktionsraum mittels einer Kühlvorrichtung gekühlt wird.

Auch hier besteht wieder der Vorteil, dass durch die Kühlung des Sorptionsmittels die Anzahl der verfügbaren Spezies erhöht wird, andererseits wird durch die Senkung der Temperatur im Reaktionsraum der Gleichgewichtsumsatz positiv beeinflusst.

Wiederum ist es auch hier zweckmäßig, dass die Kühlung des Sorptionsmittels durch die Kühlvorrichtung im Bereich der Sorptionsmittelsammelzone erfolgt.

Ferner ist es zweckmäßig, wenn das mit dem Reaktionsprodukt beladene Sorptionsmittel aus dem Reaktionsraum abgeleitet wird und das Reaktionsprodukt vom Sorptionsmittel getrennt wird. Das so entladene Sorptionsmittel kann in vorteilhafter Weise durch ein Wiedereinbringen in den Reaktionsraum rezykliert werden.

Ferner ist es vorteilhaft, wenn das beladene Sorptionsmittel im Bereich der Sorptionsmittelsammelzone durch eine Umwälzvorrichtung umgewälzt wird, um den Austausch mit der Kühlvorrichtung zu verbessern.

Weitere Ausgestaltungsformen und weitere Merkmale der Erfindung werden anhand der folgenden Figuren näher erläutert. Dabei zeigen:
Fig. 1 einen Reaktor mit einem Reaktionsraum und einer Sorptionsmittelsammelzone, wobei eine Kühlung des Sorptionsmittels in der Sorptionsmittelsammelzone erfolgt,
Fig. 2 ein Reaktor analog des Reaktors zu Fig. 1, wobei eine Kühlung des Sorptionsmittels außerhalb der Sorptionsmittelsammelzone erfolgt.

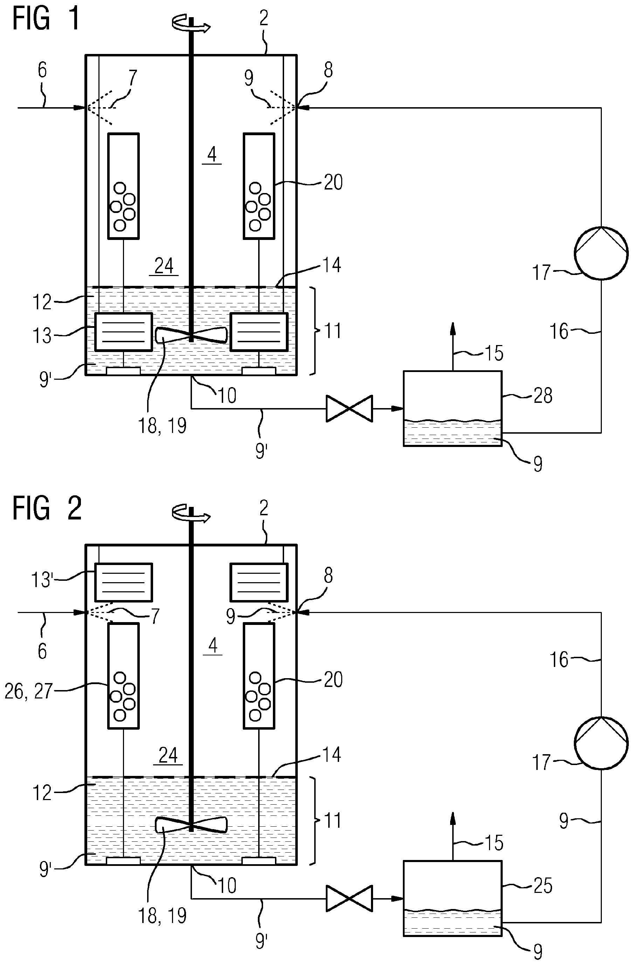

In Fig. 1 ist ein Reaktor 2, der in Form eines Rührkesselreaktors ausgestaltet ist, dargestellt. Der Reaktor 2 umfasst dabei eine Eduktezuführvorrichtung 6 sowie eine Zuführvorrichtung 8 für Sorptionsmittel 9. In einem Reaktionsraum 4 werden dabei Edukte 7 und Sorptionsmittel 9 eingebracht. Der Reaktionsraum 4 umfasst weiterhin einen Katalysatorraum 20, der dazu geeignet ist, einen Katalysator 22 aufzunehmen. Ferner umfasst der Reaktionsraum 4 einen Sorptionsraum 24, in dem sich das Sorptionsmittel 9, bevorzugt tröpfchenförmig befindet und dabei kontinuierlich in Richtung eines unteren Bereiches 11, wo sich eine Sorptionsmittelsammelzone 12 befindet, absetzt.

Im Folgenden wird dabei näher auf ein typisches Reaktionsverfahren eingegangen, das im Reaktor 2 bzw. im Reaktionsraum 4 stattfindet.

Die gasförmigen Edukte 7 werden wie bereits erwähnt, in den Reaktionsraum 4 eingeleitet, wobei sie zu der Stelle des Reaktionsraums 4 geführt werden, an dem der Katalysator 20 vorliegt. Durch das Einstellen von geeigneten Reaktionsbedingungen, die jeweils auf die entsprechende Reaktion bzw. die entsprechende Edukte ausgerichtet ist, kommt es am Katalysator zu einer exothermen (wärmetönenden) Reaktion der Edukte zu flüssigen oder gasförmigen Reaktionsprodukten 15. Dabei erhöht sich die Temperatur in einer Gasphase innerhalb des Reaktors, wodurch es zu Deaktivierungsmechanismen am Katalysator (Verkochung oder Sintern kommen kann). Die Gasphase im Reaktor umfasst dabei sowohl gasförmige Edukte 7 als auch gegebenenfalls gasförmige Reaktionsprodukte 15. In einer solchen Reaktionsanordnung werden die Edukte 7 maximal bis zum thermodynamisch möglichen Gleichgewichtsumsatz reagieren, der bei einigen Reaktionen bei niedrigen Werten liegt. Beispielsweise ist die Reaktion von Kohlendioxid und Wasserstoff zum Methanol

CO₂ + 3H₂ → CH₃OH + H₂O Gl 1

thermodynamisch durch einen niedrigen Gleichgewichtsumsatz bei typischen und wirtschaftlichen Reaktionsbedingungen limitiert. Die typischen Reaktionsbedingungen hierfür sind ein Druck von 75 bar und eine Temperatur von 250°C. Durch die starke Wärmetönung, also durch die Energiefreigabe während der Reaktion wird bei dieser Reaktion der Gleichgewichtsumsatz gesenkt, da die Reaktionstemperatur steigt. Daher ist es zweckmäßig, die durch die Reaktion der Gasphase frei werdende Wärme mittels geeigneter Maßnahmen aus dem Reaktionsraum zu entfernen. Auf die entsprechenden Maßnahmen wird noch eingegangen werden.

Als Sorptionsmittel 9 bietet sich hierbei beispielsweise eine wässrige Flüssigkeit, ein Wärmeträgeröl, eine Salzschmelze oder eine ionische Flüssigkeit an, die eine hoher Wärmekapazität aufweist und dabei einen möglichst niedrigen Dampfdruck besitzt. Eine vorteilhafte Mischung für ein Sorptionsmittel ist beispielsweise Mischung aus einem Phosphonium NTf2 IL und einem Alkali oder Erdalkali NTf2 Salz.

Eine nicht abgeschlossene Auflistung von geeigneten ionische Flüssigkeiten ist in Tabelle 1 gegeben.

**Tabelle 1: Für die Sorption geeignete Ionische Flüssigkeiten (teilweise unter Zumischung von Salzen)**

| Ionische Flüssigkeit |
|---|
| P₁₄₄₄ NTf₂ |
| P₆₆₆₁₄ NTf₂ |
| N₁₈₈₈ NTf₂ |
| Mischung aus P₁₄₄₄NTf₂ und Li NTf₂ |
| Mischung aus P₁₄₄₄NTf₂ und Cs NTf₂ |
| Mischung aus P₁₄₄₄NTf₂ und Mg (NTf₂)₂ |
| Mischung aus P₁₄₄₄NTf₂ und Ca (NTf₂) |
| P₁₄₄₄ MeSO₄ |
| P₁₄₄₄ Me₂PO₄ |
| EMIM NTf₂ |
| EMIM MeSO₃ |
| P₁₄₄₄ OTf |
| P₂₄₄₄ Et₂PO₄ |

Das Sorptionsmittel kann die in der Gasphase enthaltenen Wärme aufnehmen und somit die Temperatur der Gasphase absenken. Dies führt zu einer homogenen Temperaturverteilung in der Reaktionszone (im Umfeld des Katalysators) und damit idealerweise zu einem isothermen Verhalten im Reaktionsraum 4. Hierzu ist ein effektiver Wärmeaustausch der Gasphase, also insbesondere der Phase der Edukte 7, mit dem Sorptionsmittel 9 erforderlich.

Der Katalysator 21 und das Sorptionsmittel 9 sind Stoffe, die in einigen Fällen möglichst nicht miteinander in Berührung kommen sollen, da dadurch ihre Stabilität und ihre Funktionalität negativ beeinflusst werden können. Aus diesem Grund ist es zweckmäßig, dass der Katalysatorraum 20 vom Sorptionsraum 24 durch ein gasdurchlässiges Element 26 getrennt ist. Dieses gasdurchlässige Element 26 kann beispielsweise in Form eines metallischen Gewebes oder in Form einer gegenüber den entsprechenden Gasphasen durchlässigen Membran ausgebildet sein. Dies ermöglicht es, dass die in der Regel gasförmigen Edukte 7 durch das gasdurchlässige Element 26 durchdringen und an der Katalysatoroberfläche zu den Reaktionsprodukten 15 reagieren. Die Reaktionsprodukte 15 sind bei der vorherrschenden Atmosphäre ebenfalls in der Regel gasförmig und verlassen den Katalysatorraum 20 durch das entsprechende Element 26. Nachdem sie durch das Element 26 in den Sorptionsraum 24 eingetreten sind, können sie vom Sorptionsmittel 9 sorbiert werden. Das mit den Reaktionsprodukten 15 beladene Sorptionsmittel 9 wird im Weiteren als 9' bezeichnet.

In Fig. 1 ist dabei eine Ausführungsform gezeigt, wobei das Sorptionsmittel direkt innerhalb des Reaktionsraumes 4 in einer Reaktionsmittelsammelzone 12 durch eine Kühlvorrichtung 13 gekühlt wird. Die Kühlvorrichtung 13 ist dabei so ausgestaltet, dass sie unterhalb eines Flüssigkeitsspiegels 14 der Sorptionsmittelsammelzone 12 angeordnet ist. Die Kühlvorrichtung 13 ist somit vollständig in das Sorptionsmittel in der Sammelzone 12 eingetaucht, was zu einer sehr effektiven Kühlung des Sorptionsmittels 9 führt. Ferner ist es zweckmäßig, eine Umwälzvorrichtung 18, beispielsweise in Form einer Rührvorrichtung 19 einzubringen. Dies bewirkt, dass das erwärmte und mit den Reaktionsprodukten 15 beladenen Sorptionsmittel 9' im Bereich der Sorptionsmittelsammelzone 12 kontinuierlich um die Kühlvorrichtung 13 fließt und somit ein optimaler Wärmeaustausch stattfindet.

Als Kühlvorrichtung 13 können Wärmetauscher verwendet werden, die in Form von Kassetten in der Sorptionsmittelsammelzone 12 angeordnet sind. Grundsätzlich sind jedoch auch andere Ausgestaltungsformen zweckmäßig, beispielsweise kann eine Kühlvorrichtung 13 direkt in einer Wand des Reaktionsraums 4 integriert sein.

Diese Ausgestaltungsform hat den Vorteil, dass die Wärme direkt im Reaktor 2 bzw. im Reaktionsraum 4 abgeführt werden kann und es im Reaktionsraum 4 nicht zu einer Erwärmung und damit zu einer verminderten Aufnahmefähigkeit des Sorptionsmittels 9 für Reaktionsprodukte 15 kommt. Anschließend wird ein Teil des mit dem Reaktionsprodukt 15 beladenen Sorptionsmittels 9' aus der Reaktionsmittelsammelzone 12 über eine Ablassvorrichtung 10 aus dem Reaktionsraum 4 entfernt. Das beladene Sorptionsmittel 9' wird in einen Phasenabscheider 28 geführt, in dem das Sorptionsmittel 9 vom Produkt 15 getrennt wird. Das Produkt 15 wird abgezogen und anderweitig gesammelt. Das entladene Sorptionsmittel 9 wird über eine Rezykliervorrichtung 16, die eine Pumpe 17 umfasst, wieder der Zuführvorrichtung 8 für das Sorptionsmittel 9 zugeführt. Das auf diese Weise rezyklierte Sorptionsmittel 9 gelangt wieder in den Reaktionsraum 4 und ist bereit zur Aufnahme von Reaktionsprodukten 15.

In einer weiteren Ausgestaltungsform gemäß Fig. 2 ist die Kühlvorrichtung 13' nicht wie in Fig. 1 dargestellt, im Bereich der Sorptionsmittelsammelzone 12 angeordnet, sondern in einem oberen Bereich des Reaktionsraumes 4. Hierin unterscheiden sich die ansonsten identischen Darstellungen von Fig. 1 und Fig. 2. Die an dieser Stelle angeordnete Kühlvorrichtung 13' fördert das Lösen von Reaktionsprodukten 15 in dem Sorptionsmittel 9 und fördert ebenfalls die Kondensation der flüssigen Produkte 15. Auch hierdurch tritt eine Kühlung des Reaktionsraumes 4 und somit der gesamten atmosphärischen Umgebung im Reaktionsraum 4 ein, was sich positiv auf die Reaktionsbedingungen und somit auf den Gleichgewichtsumsatz auswirkt. Bevorzugt werden die Edukte 7 gasförmig in den Reaktionsraum 4 eingebracht, es ist jedoch auch alternativ möglich, die Edukte 7 in einer kondensierten Phase, also in flüssiger Form in den Reaktionsraum 4 einzubringen. Edukte 7 werden dabei gezielt in einmem flüssigen bzw. überkritischen Zustand bei einer hohen Fluiddichte dem Reaktor 2 zugeführt. Im Reaktor findet dann im Reaktionsraum 4 eine Verdampfung statt, da die Edukte bzw. das Edukt unter Reaktionsbedingungen gasförmig ist. Die flüssigen Edukte können gezielt an den Ort der Wärmefreisetzung am Katalysator 22 eingebracht werden. Hierbei wird eine gezielte lokale Eindüsung, also eine entsprechende, hier nicht dargestellte Ausgestaltungsform der Zuführvorrichtung 6 für Edukte 7 angewandt. Bei der Reaktion gemäß Gleichung 1 zur Synthese von Methanol würde in diesem Fall bevorzugt das Edukt Kohlendioxid in flüssiger bzw. überkritischer Form zugegeben werden.

## Patentansprüche

1. Verfahren zum Betreiben eines Reaktors zur Umsetzung gleichgewichtslimitierter Reaktionen, umfassend folgende Verfahrensschritte,
Einbringen von Edukten (7) in einen Reaktionsraum (4), Einbringen eines flüssigen ionischen Sorptionsmittels (9) in den Reaktionsraum (4),
wobei die Edukte (7) an einen im Reaktionsraum (4) befindlichen Katalysator (22) geführt werden und an einer Katalysatoroberfläche bis zum Auftreten einer Gleichgewichtssituation zu Reaktionsprodukten (15) umgesetzt werden, wobei die Reaktionsprodukte (15) von der Katalysatoroberfläche zum Sorptionsmittel (9) gelangen und von diesem sorbiert werden, wonach sich das mit den Reaktionsprodukten (15) beladene Sorptionsmittel (9') in einer Sorptionsmittelsammelzone absetzt, **dadurch gekennzeichnet, dass** das flüssige Sorptionsmittel (9, 9') im Reaktionsraum mittels einer Kühlvorrichtung gekühlt wird.

2. Verfahren nach Anspruch 1, **dadurch gekennzeichnet, dass** das Sorptionsmittel (9, 9') im Bereich der Sorptionsmittelsammelzone (12) durch die Kühlvorrichtung (13) gekühlt wird.

3. Verfahren nach Anspruch 1 oder 2, **dadurch gekennzeichnet, dass** das mit dem Reaktionsprodukt (15) beladene Sorptionsmittel (9') aus dem Reaktionsraum (4) abgeleitet wird und das Reaktionsprodukt (15) vom Sorptionsmittel (9') getrennt wird und das so entladene Sorptionsmittel (9) durch Wiedereinbringen in den Reaktionsraum (4) rezykliert wird.

4. Verfahren nach einem der Ansprüche 1 bis 3, **dadurch gekennzeichnet, dass** das beladen Sorptionsmittel (9') im Bereich der Sorptionsmittelsammelzone (12) durch eine Umwälzvorrichtung (18) umgewälzt wird.

5. Verwendung einer ionischen Flüssigkeit als Sorbtionsmittel in einem Verfahren gemäß einem der Ansprüche 1 bis 4 zur kontinuierlichen Abführung von Reaktionsprodukten aus einer gleichgewichtslimitierten Reaktion, wobei die ionische Flüssigkeit eine oder mehrere Bestandteile aus folgender Liste umfasst:
| |
|---|
| P₁₄₄₄ NTf₂ |
| P₆₆₆₁₄ NTf₂ |
| N₁₈₈₈ NTf₂ |
| Mischung aus P₁₄₄₄NTf₂ und Li NTf₂ |
| Mischung aus P₁₄₄₄NTf₂ und Cs NTf₂ |
| Mischung aus P₁₄₄₄NTf₂ und Mg (NTf₂) |
| Mischung aus P₁₄₄₄NTf₂ und Ca (NTf₂) |
| P₁₄₄₄ MeSO₄ |
| P₁₄₄₄ Me₂PO₄ |
| EMIM NTf₂ |
| EMIM MeSO₃ |
| P₁₄₄₄ OTf |
| P₂₄₄₄ Et₂PO₄ |

## Claims

1. Process for operating a reactor for conversion of equilibrium-limited reactions comprising the process steps of
introducing reactants (7) into a reaction space (4), introducing a liquid ionic sorbent (9) into the reaction space (4),
wherein the reactants (7) are passed over a catalyst (22) located in the reaction space (4) and are converted into reaction products (15) over a catalyst surface until attainment of an equilibrium situation, wherein the reaction products (15) pass from the catalyst surface to the sorbent (9) and are sorbed thereby, after which the sorbent (9') laden with the reaction products (15) settles in a sorbent collection zone, **characterized in that** the liquid sorbent (9, 9') is cooled by means of a cooling apparatus in the reaction space.

2. Process according to Claim 1, **characterized in that** the sorbent (9, 9') is cooled by the cooling apparatus (13) in the region of the sorbent collection zone (12).

3. Process according to Claim 1 or 2, **characterized in that** the sorbent (9') laden with the reaction product (15) is discharged from the reaction space (4) and the reaction product (15) is separated from the sorbent (9') and the thus stripped sorbent (9) is recycled by reintroduction into the reaction space (4) .

4. Process according to any of Claims 1 to 3, **characterized in that** the laden sorbent (9') is circulated by a circulation apparatus (18) in the region of the sorbent collection zone (12).

5. Use of an ionic liquid as a sorbent in a process according to any of Claims 1 to 4 for continuous removal of reaction products from an equilibrium-limited reaction, wherein the ionic liquid comprises one or more constituents from the following list:
| |
|---|
| P₁₄₄₄ NTf₂ |
| P₆₆₆₁₄ NTf₂ |
| N₁₈₈₈ NTf₂ |
| Mixture of P₁₄₄₄ NTf₂ and Li NTf₂ |
| Mixture of P₁₄₄₄ NTf₂ and Cs NTf₂ |
| Mixture of P₁₄₄₄ NTf₂ and Mg (NTf₂)₂ |
| Mixture of P₁₄₄₄ NTf₂ and Ca (NTf₂)₂ |
| P₁₄₄₄ MeSO₄ |
| P₁₄₄₄ Me₂PO₄ |
| EMIM NTf₂ |
| EMIM MeSO₃ |
| P₁₄₄₄ OTf |
| P₂₄₄₄ Et₂PO₄ |

## Revendications

1. Procédé pour faire fonctionner un réacteur pour effectuer des réactions limitées à l'équilibre, comprenant les stades de procédé suivants,
introduction d'éduits (7) dans un espace (4) de réaction, introduction d'un agent (9) de sorption ionique liquide dans l'espace (4) de réaction,
les éduits (7) passant sur un catalyseur (22) se trouvant dans l'espace (4) de réaction et étant transformés en produits (15) de réaction sur une surface du catalyseur jusqu'à l'apparition d'une situation d'équilibre, les produits (15) de réaction arrivant de la surface du catalyseur à l'agent (9) de sorption et étant sorbés par celui-ci, l'agent (9') de sorption, chargé des produits (15) de réaction, se déposant dans une zone de recueil de l'agent de sorption,
**caractérisé en ce que** l'on refroidit l'agent (9, 9') de sorption liquide dans l'espace de réaction au moyen d'un système de refroidissement.

2. Procédé suivant la revendication 1, **caractérisé en ce que** l'on refroidit l'agent (9, 9') de sorption par le système (13) de refroidissement dans la région de la zone (12) de recueil de l'agent de sorption.

3. Procédé suivant la revendication 1 ou 2, **caractérisé en ce que** l'on fait sortir l'agent (9') de sorption, chargé du produit (15) de réaction, de l'espace (4) de réaction et **en ce que** l'on sépare le produit (15) de réaction de l'agent (9') de sorption et on recycle l'agent (9) de sorption déchargé en le réintroduisant dans l'espace (4) de réaction.

4. Procédé suivant l'une des revendications 1 à 3, **caractérisé en ce que** l'on fait circuler l'agent (9') de sorption chargé, par un système (18) de recirculation, dans la région de la zone (12) de recueil de l'agent de sorption.

5. Utilisation d'un liquide ionique comme agent de sorption dans un procédé suivant l'une des revendications 1 à 4 pour l'évacuation en continu de produits de réaction d'une réaction limitée à l'équilibre, le liquide ionique comprenant un ou plusieurs de la liste suivante :
P₁₄₄₄ NTf₂
P₆₆₆₁₄ NTf₂
N₁₈₈₈ NTf₂
mélange de P₁₄₄₄NTf₂ et Li NTf₂
mélange de P₁₄₄₄NTf₂ et Cs NTf₂
mélange de P₁₄₄₄NTf₂ et Mg(NTf₂)₂
mélange de P₁₄₄₄NTf₂ et Ca(NTf₂)₂
P₁₄₄₄ MeSO₄
P₁₄₄₄ Me₂PO₄
EMIM NTf₂
EMIM MeSO₃
P₁₄₄₄ OTf
P₂₄₄₄ Et₂PO₄
